# EUROPEAN PATENT APPLICATION

(11) **EP 2 762 054 A1**
(43) Date of publication of application: **06.08.2014**
(21) Application number: 12835042.8
(22) Date of filing: 28.09.2012
(51) Int. Cl.: A61B 1/00

(54) **TREATMENT DEVICE AND ENDOSCOPIC TREATMENT SYSTEM**

(30) Priority: 30.09.2011 US 201161541585 P
(71) Applicant: Olympus Medical Systems Corp., Tokyo 151-0072 (JP)
(72) Inventor: YAMATANI, Ken, Tokyo 151-0072 (JP); KAJI, Kunihide, Tokyo 151-0072 (JP)
(74) Representative: Gunzelmann, Rainer
(86) International application number: PCT/JP2012/075025
(87) International publication number: WO 2013/047723

(57) **Abstract**

An endoscopic device inserted into a channel in an endoscope, the endoscopic device includes a curved area being protruded from an opening of a distal end of the channel; an elongated flexible longitudinal shaft member having a rotation guide member located in the curved area, and a treatment unit installed at a distal end section of the longitudinal shaft member. A curve direction of the curved area and a curve direction of the rotation guide section are opposite to each other.

## Description

### [Field of the Invention]

The present invention relates to an endoscopic device and an endoscope treatment system.

Priority is claimed on US Provisional Patent Application No. 61/541,585, filed September 30, 2011, the contents of which are incorporated herein by reference.

### [Description of Related Art]

An operator of an endoscope guides an endoscopic device attached to the endoscope to a treatment target to treat the treatment target using the endoscopic device.

In Patent Document 1, an endoscope having a cylindrical guide tool having a curved section that is bent in an arc shape is disclosed. The arc-shaped curved section of the guide tool is inserted into a channel of the endoscope. The operator can rotate the arc-shaped curved section of the guide tool in the curved section of the endoscope. An orientation of the guide tool is held in a state in which curve directions of the curved section of the endoscope and the arc-shaped curved section of the guide tool coincide with each other.

### [Prior Art Document]

### [Patent Document]

Patent Document 1: Japanese Unexamined Patent Application, First Publication No. H09-299323

### [Summary of the Invention]

### [Means for Solving the Problems]

An endoscopic device according to a first aspect of the present invention, which is inserted into a channel in an endoscope, the endoscope including an observation unit being used to observe a treatment target, a curve section which is capable of being curvedly operated, an insertion section which is an elongated member having a distal end and a proximal end and in which the observation unit and the curve section are disposed at the distal end, the insertion section being inserted into a body, and a channel of an endoscope formed inside the insertion section. The endoscopic device including: an elongated flexible longitudinal shaft member which is capable of freely advancing and retreating inside the channel; a treatment unit installed at a distal end section of the longitudinal shaft member and performs a treatment of the treatment target; a curved area which is a first portion of the longitudinal shaft member and being protruded from an opening of a distal end of the channel in a use state in which the longitudinal shaft member is inserted into the channel; and a rotation guide section which is a second portion of the longitudinal shaft member and disposed at inside the channel and the curve section in a use state. A curve direction of the curved area and a curve direction of the rotation guide section are opposite to each other.

The endoscopic device according to a second aspect of the present invention, in the endoscopic device according to the first aspect, in a process in which the longitudinal shaft member is moved from a proximal end side to a distal end side of the longitudinal shaft member in the channel when the curve section is in a curved state, the rotation guide section may be rotated about a central axis of the channel as a rotational center, the curved area may be rotated such that the treatment unit approaches an optical axis of the observation unit by rotating the rotation guide section, and an orientation of the curved area may be held in a positional relationship in which the treatment unit is proximate to the optical axis in the use state.

The endoscopic device according to a third aspect of the present invention, in the endoscopic device according to the first aspect, the curved area of the longitudinal shaft member may be moved in a direction away from an optical axis of the observation unit by causing curvature in the curve section of the endoscope and being protruded the rotation guide section from the opening of the distal end of the channel.

The endoscopic device according to a sixth aspect of the present invention, in the endoscopic device according to the first aspect, the rotation guide section may be a curbed shape area of the longitudinal shaft member having a curved bias in a predetermined direction.

The endoscopic device according to a fifth aspect of the present invention, in the endoscopic device according to the first aspect, the rotation guide section may be an area in which at least a portion of the longitudinal shaft member is formed at one of a rectangular shape, an elliptical shape, and an oval shape of an outline of a cross-section perpendicular to the longitudinal shaft.

The endoscopic device according to a sixth aspect of the present invention, in the endoscopic device according to the first aspect, a lumen may be formed at the longitudinal shaft member along a longitudinal direction thereof, an opening part which is communicated with the lumen of the longitudinal shaft member and allows a liquid in the body to enter the lumen, may be formed at the treatment unit, and a suction mechanism in which the liquid is moved from an inside the body to an outside of the body via the opening part and the lumen, may be capable of connecting to the longitudinal shaft member.

An endoscopic treatment system according to a first aspect of the present invention includes an endoscope and an endoscopic device. The endoscope includes: an observation unit being used to observe a treatment target; a curve section which is capable of being curvedly operated; an insertion section which is an elongated member having a distal end and a proximal end and in which the observation unit and the curve section are disposed at the distal end inserted into a body; and a channel of an endoscope formed inside the insertion section. The endoscopic device includes: an elongated flexible longitudinal shaft member which is capable of freely advancing and retreating inside the channel; a treatment unit installed at a distal end section of the longitudinal shaft member and performs a treatment of the treatment target; a curved area which is a first portion of the longitudinal shaft member and being protruded from an opening of a distal end of the channel in a use state in which the longitudinal shaft member is inserted into the channel; and a rotation guide section which is a second portion of the longitudinal shaft member and disposed at inside the channel and the curve section in a use state. A curve direction of the curved area and a curve direction of the rotation guide section are opposite to each other.

The endoscopic treatment system according to a second aspect of the present invention, in the endoscopic treatment system according to the first aspect, in a process in which the longitudinal shaft member is moved from a proximal end side to the distal end side of the longitudinal shaft member in the channel, the rotation guide section may be rotated about a central axis of the channel as a rotational center such that the treatment unit approaches an optical axis of the observation unit, and an orientation of the curved area may be held in a positional relationship in which the treatment unit is proximate to the optical axis in the use state.

The endoscopic treatment system according to a third aspect of the present invention, in the endoscopic treatment system according to the first aspect, the endoscope may include: a manipulation unit disposed at a proximal end of the insertion section and configured to input a manipulation for curvedly operating the curve section; a power transmission member connected to the manipulation unit and the curve section and configured to transmit a manipulation power from the manipulation unit to the curve section; and a curve lock mechanism configured to hold the curve section in a curved state of a predetermined curvature, wherein the rotating guide section may be a curved shaped area of the longitudinal shaft member having a curved bias corresponding to the predetermined curvature.

### [Brief Description of the Drawings]

FIG. 1 is a general view showing an endoscope treatment system of an embodiment of the present invention.
FIG. 2 is a front view showing an insertion section of an endoscope.
FIG. 3 is a general view showing an endoscopic device of the embodiment of the present invention.
FIG. 4 is a view for describing a process of the endoscope treatment system in use.
FIG. 5 is a view for describing the process of the endoscope treatment system in use.
FIG. 6 is a front view showing the insertion section of the endoscope in a first use state.
FIG. 7 is a photograph showing the endoscope in the first use state.
FIG. 8 is an endoscope image in the first use state.
FIG. 9 is a front view showing the insertion section of the endoscope in a second use state.
FIG. 10 is a photograph showing the endoscope in the second use state.
FIG. 11 is an endoscope image in the second use state.
FIG. 12 is a front view showing the insertion section of the endoscope in a third use state.
FIG. 13 is a photograph showing the endoscope in the third use state.
FIG 14 is an endoscope image in the third use state.
FIG. 15 is a front view showing the insertion section of the endoscope in a fourth use state.
FIG. 16 is a photograph showing the endoscope in the fourth use state.
FIG. 17 is an endoscope image in the fourth use state.
FIG. 18 is a front view showing the insertion section of the endoscope in a fifth use state.
FIG. 19 is a photograph showing the endoscope in the fifth use state.
FIG. 20 is an endoscope image in the fifth use state.
FIG. 21 is a front view showing the insertion section of the endoscope in a sixth use state.
FIG. 22 is a photograph showing the endoscope in the sixth use state.
FIG. 23 is an endoscope image in the sixth use state.
FIG. 24 is a front view showing the insertion section of the endoscope in a seventh use state.
FIG. 25 is a photograph showing the endoscope in the seventh use state.
FIG. 26 is an endoscope image in the seventh use state.
FIG. 27 is a view for describing a process of the endoscope treatment system in use.
FIG. 28 is a view showing Positional Relationship 1 between a curved area and a rotation guide section.
FIG. 29 is a view showing Positional Relationship 2 between the curved area and the rotation guide section.
FIG. 30 is a view showing Positional Relationship 3 between the curved area and the rotation guide section.
FIG. 31 is a cross-sectional view showing Modified example 1 (a rotation guide section 6A) of the rotation guide section.
FIG. 32 is a cross-sectional view showing Modified example 2 (a rotation guide section 6B) of the rotation guide section.
FIG. 33 is a cross-sectional view showing Modified example 3 (a rotation guide section 6C) of the rotation guide section.
FIG. 34 is a perspective view showing Modified example 1 (a treatment unit 3A) of the endoscopic device.
FIG. 35 is a perspective view showing Modified example 2 (a treatment unit 3B) of the endoscopic device.
FIG. 36 is a perspective view showing Modified example 3 (a treatment unit 3C) of the endoscopic device.
FIG. 37 is a perspective view showing Modified example 4 (a treatment unit 3D) of the endoscopic device.
FIG. 38 is a perspective view showing Modified example 5 (a treatment unit 3E) of the endoscopic device.
FIG. 39 is a perspective view showing Modified example 6 (a treatment unit 3F) of the endoscopic device.
FIG 40 is a perspective view showing Modified example 7 (a treatment unit 3G) of the endoscopic device.
FIG. 41 is a perspective view showing Modified example 8 (a treatment unit 3H) of the endoscopic device.
FIG. 42 is a cross-sectional view taken along line B-B of FIG. 41.
FIG. 43 is a cross-sectional view taken along line C-C of FIG. 41.
FIG 44 is a perspective view showing Modified example 9 (a treatment unit 3I) of the endoscopic device.
FIG. 45 is a perspective view showing a modified example (an endoscope treatment system 100A) of the endoscope treatment system.

### [Description of Embodiments]

An endoscopic device 1 and an endoscope treatment system 100 of an embodiment of the present invention will be described.

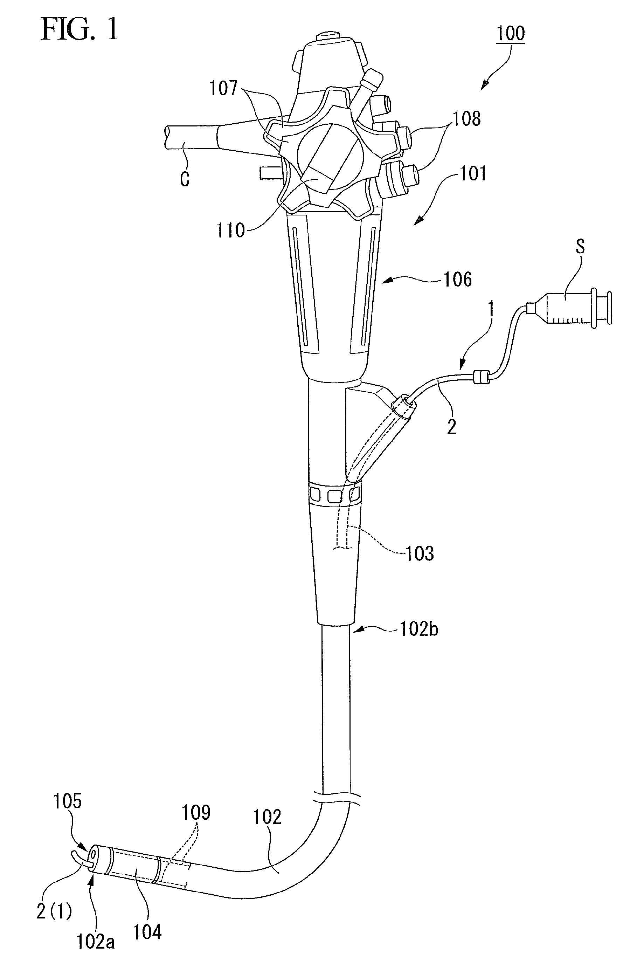

FIG 1 is a general view of the endoscope treatment system 100 of the embodiment of the present invention.

As shown in FIG. 1, the endoscope treatment system 100 includes an endoscope 101 and the endoscopic device 1. The endoscopic device 1 is combined with the endoscope 101 and used.

A configuration of the endoscope 101 will be described.

As shown in FIG. 1, the endoscope 101 includes an insertion section 102, a curve section 104, an observation unit 105, a manipulation unit 106, a power transmission member 109, and a curve lock mechanism 110.

The insertion section 102 has a distal end 102a and a proximal end 102b.

The curve section 104 is disposed at the distal end 102a of the insertion section 102.

The observation unit 105 is disposed at a distal end of the curve section 104.

The manipulation unit 106 is disposed at a proximal end of the insertion section 102.

The power transmission member 109 is connected to the manipulation unit 106 and the curve section 104.

The curve lock mechanism 110 is installed at the manipulation unit 106.

The insertion section 102 is an elongated member having flexibility. The insertion section 102 is inserted into the body. A cylindrical channel 103 configured to guide the endoscopic device 1 to a treatment target is formed in the insertion section 102.

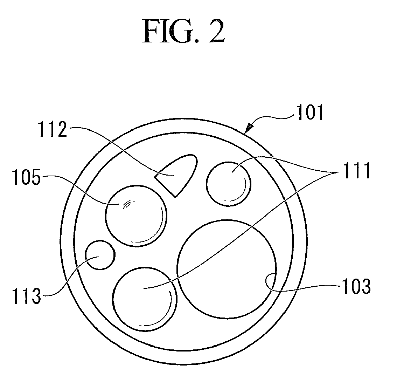

As shown in FIG. 2, a distal end of the channel 103 is opened at a distal end surface of the insertion section 102.

A light guide 111, a nozzle 112 and a water supply pipe 113 are disposed at the distal end surface of the insertion section 102.

The light guide 111 radiates illumination light. The nozzle 112 sprays liquid to clean the observation unit 105.

As shown in FIG. 1, the curve section 104 is a cylindrical member concentrically installed on a central axis of the insertion section 102. The curve section 104 includes a plurality of knob rings or curved tips (hereinafter referred to as "knob rings or the like") connected along the central axis of the insertion section 102. Each of the knob rings or the like is formed in an annular shape. The neighboring knob rings or the like are pivotable about a connecting area thereof. For this reason, the curve section 104 can be curvedly operated as a whole.

The observation unit 105 is used to observe the treatment target. The observation unit 105 has an object optical system, an image sensor and a driver circuit. The observation unit 105 can photograph the treatment target.

The manipulation unit 106 is an area at which various manipulations are performed. The manipulation unit 106 is gripped by an operator upon use of the endoscope 101. The operator can curvedly operate the curve section 104, and perform air supply, water supply, and suction thereof by manipulating the manipulation unit 106.

A knob 107, a switch 108, and a connection port for a universal cable C are installed at the manipulation unit 106.

The curve section 104 can be curvedly operated by manipulation of the knob 107.

Operations of the air supply, water supply and suction can be controlled by manipulation of the switch 108.

An image signal from the observation unit 105 can be output to the outside and electrical power for driving the endoscope 101 can be supplied to the endoscope 101 via the connection port of the universal cable C.

The power transmission member 109 transmits manipulation power from the manipulation unit 106 to the curve section 104. The power transmission member 109 of the embodiment is, for example, a plurality of wires formed of stainless steel.

One of two end sections of the power transmission member 109 is fixed to the knob ring or the like disposed at the distal end portion of the curve section 104. The other of the two end sections is connected to the knob 107.

When the operator rotates the knob 107, the power transmission member 109 is pulled. Accordingly, the curve section 104 fixed to the power transmission member 109 is curvedly operated.

The curve lock mechanism 110 holds the curve section 104 in a curved state in which a predetermined curvature is achieved. The curve lock mechanism 110 of the embodiment fixes a position of the knob 107 with respect to the manipulation unit 106 at a predetermined holding position within an operational range of the knob 107.

The operational range of the knob 107 may be preset according to an area in which the endoscope treatment system 100 is applied, procedure content, or the like.

A configuration of the endoscope 101 is not limited to the above-mentioned configuration but the endoscope 101 including a known configuration may be appropriately selected and employed.

Next, a configuration of the endoscopic device 1 will be described.

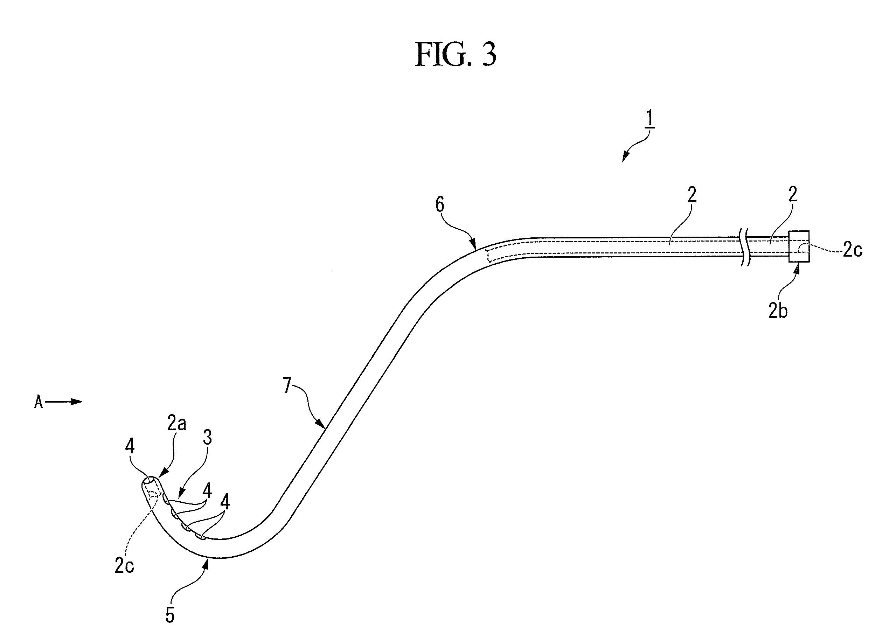

FIG. 3 is a general view of the endoscopic device 1 of the embodiment of the present invention. The endoscopic device 1 is installed at the endoscope treatment system 100.

As shown in FIG. 3, the endoscopic device 1 includes a longitudinal shaft member 2, a treatment unit 3, a curved area 5 and a rotation guide section 6. The curved area 5 and the rotation guide section 6 are formed at a portion of the longitudinal shaft member 2.

The longitudinal shaft member 2 is an elongated flexible cylindrical member. The longitudinal shaft member 2 can advance or retreat inside the channel 103 (see FIG. 1).

A lumen 2c is formed at the longitudinal shaft member 2 along a longitudinal direction thereof. A length dimension of the longitudinal shaft member 2 is larger than a length dimension of the channel 103 of the insertion section 102.

The treatment unit 3 is installed at one end section (a distal end section) of the longitudinal shaft member 2. The treatment unit 3 performs elimination treatment of liquid in the body to the outside of the body or collection treatment of the liquid in the body.

The treatment unit 3 has openings 4 in communication with the lumen 2c of the longitudinal shaft member 2. The opening 4 allows the liquid in the body to enter the lumen 2c. The openings 4 are formed at a plurality of places of an end surface of a forefront of the longitudinal shaft member 2 and an outer circumferential surface of the distal end side of the longitudinal shaft member 2.

In the treatment unit 3 of the embodiment, the plurality of openings 4 formed at the outer circumferential surface of the distal end side of the longitudinal shaft member 2 are disposed at an inner side surface curved in the curved area 5.

The curved area 5 is a portion of the longitudinal shaft member 2. The curved area 5 has a curved bias. That is, the curved area 5 has a curved shape in a state in which no external force is applied to the endoscopic device 1 (hereinafter referred to as "an unloaded state").

The curved shape of the curved area 5 may be an arc shape having a certain curvature or a smoothly curved shape in which a curvature is gradually varied. A central axis of the longitudinal shaft member 2 in the curved area 5 is disposed on the same plane in the unloaded state.

A curvature and a dimension of the curved area 5 are preset such that the treatment unit 3 is disposed within an imaging field of vision of the observation unit 105. The curvature and dimension of the curved area 5 are set based on a distance between the observation unit 105 and the channel 103, a width of the imaging field of vision of the observation unit 105, a focal distance of an object optical system in the observation unit 105, or the like.

The rotation guide section 6 is a portion of the longitudinal shaft member 2. The rotation guide section 6 is disposed inside the curve section 104 in a use state of the endoscopic device 1. The rotation guide section 6 is a curved shape area of the longitudinal shaft member 2 having a curved bias in a predetermined direction.

The curvature of the rotation guide section 6 of the embodiment is equal to a predetermined curvature of the curve section 104 of the endoscope 101.

The curvature of the rotation guide section 6 indicates a curvature in the unloaded state. A curve direction of the curved area 5 and a curve direction of the rotation guide section 6 are opposite to each other. In the unloaded state, the distal end section of the endoscopic device 1 has a curved bias in an S shape as a whole.

A linear section 7 is formed between the curved area 5 and the rotation guide section 6. The linear section 7 is a portion of the longitudinal shaft member 2. The linear section 7 is a linear area in the longitudinal shaft member 2.

An upper limit of the dimension of the linear section 7 may be a dimension such that focus of the image imaged by the observation unit 105 is set on the treatment unit 3. Here, the rotation guide section 6 is disposed at the curve section 104 of the endoscope 101.

Next, operations of the endoscopic device 1 and the endoscope treatment system 100 will be described.

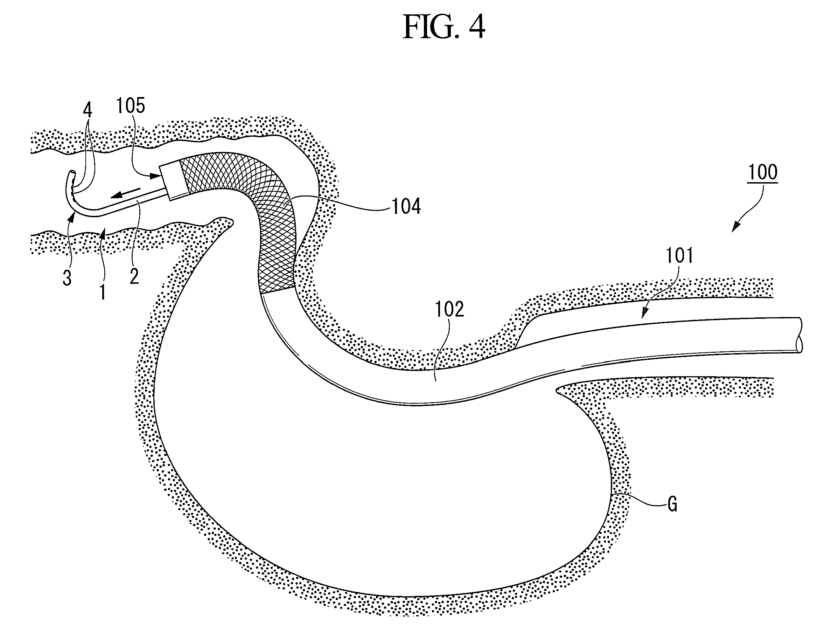

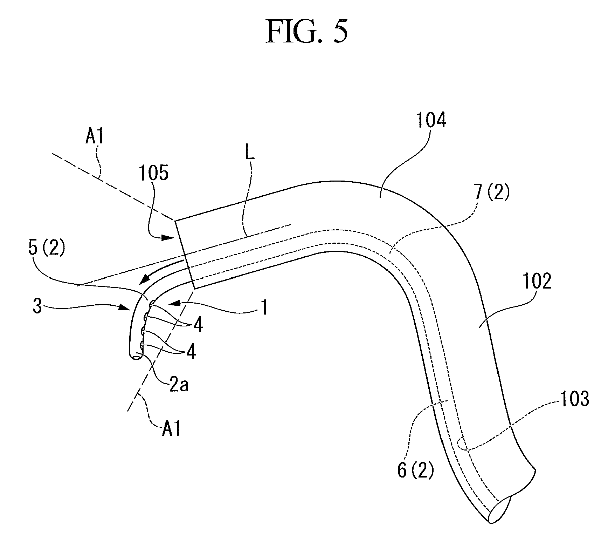

FIGS. 4 and 5 are views for describing a process of the endoscope treatment system 100 in use.

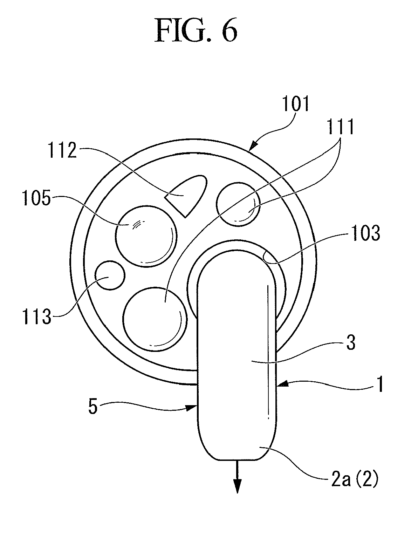

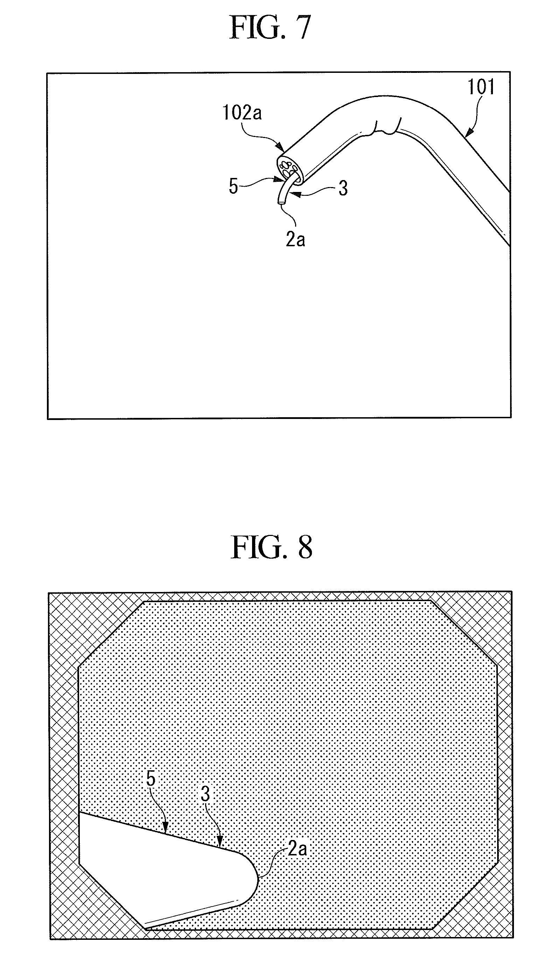

FIGS. 6 to 8 are views showing a first use state of the endoscope treatment system 100. FIG. 6 is a front view of the insertion section of the endoscope in the first use state. FIG 7 is a photograph showing the endoscope in the first use state. FIG. 8 is an endoscope image in the first use state.

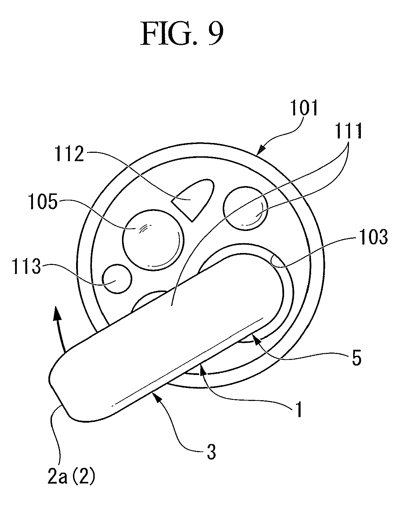

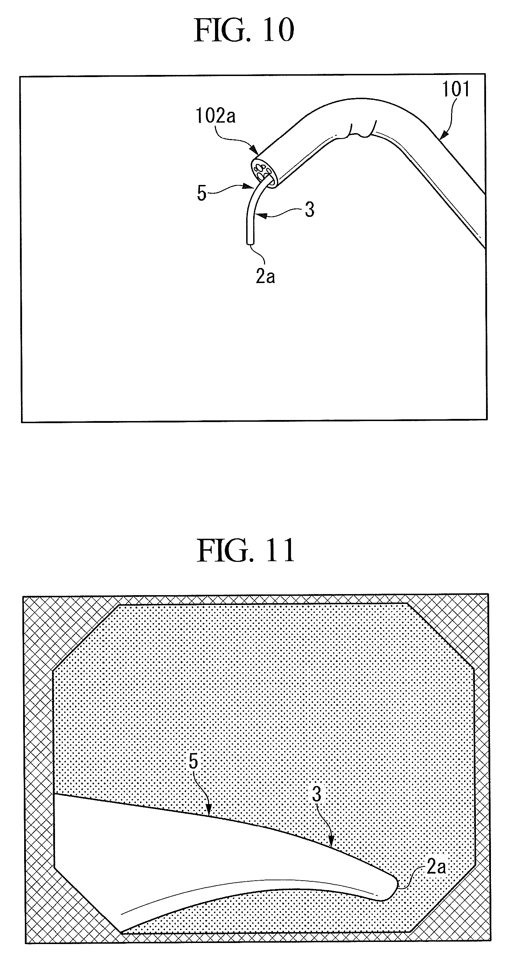

FIGS. 9 to 11 are views showing a second use state of the endoscope treatment system 100. FIG. 9 is a front view of the insertion section of the endoscope in the second use state. FIG. 10 is a photograph showing the endoscope in the second use state. FIG. 11 is an endoscope image in the second use state.

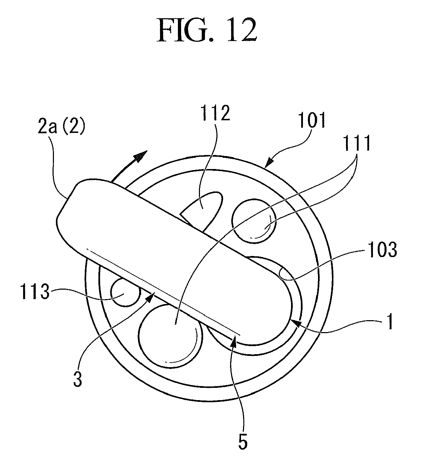

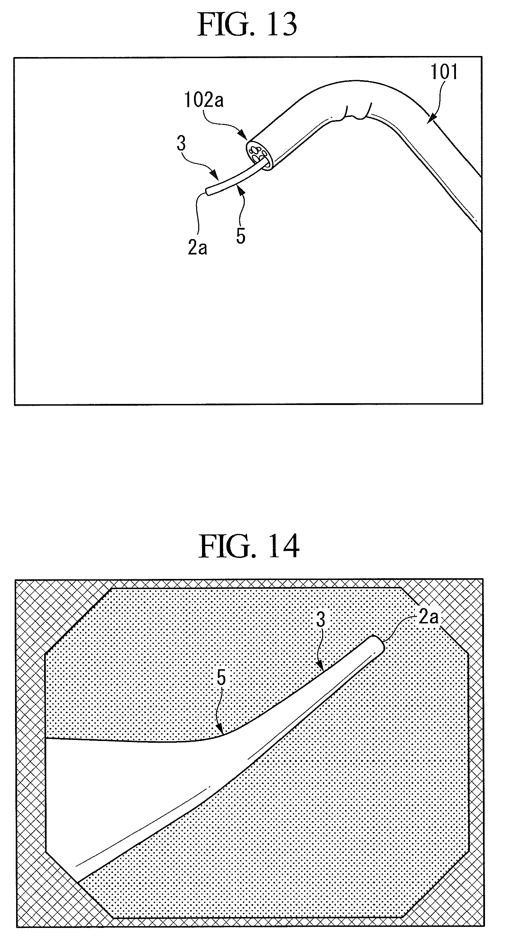

FIGS. 12 to 14 are views showing a third use state of the endoscope treatment system 100. FIG. 12 is a front view showing the insertion section of the endoscope in the third use state. FIG. 13 is a photograph showing the endoscope in the third use state. FIG. 14 is an endoscope image in the third use state.

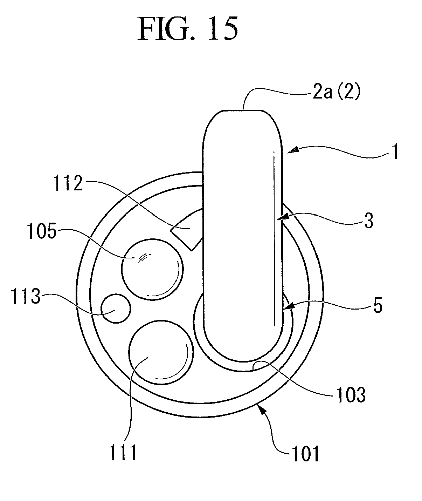

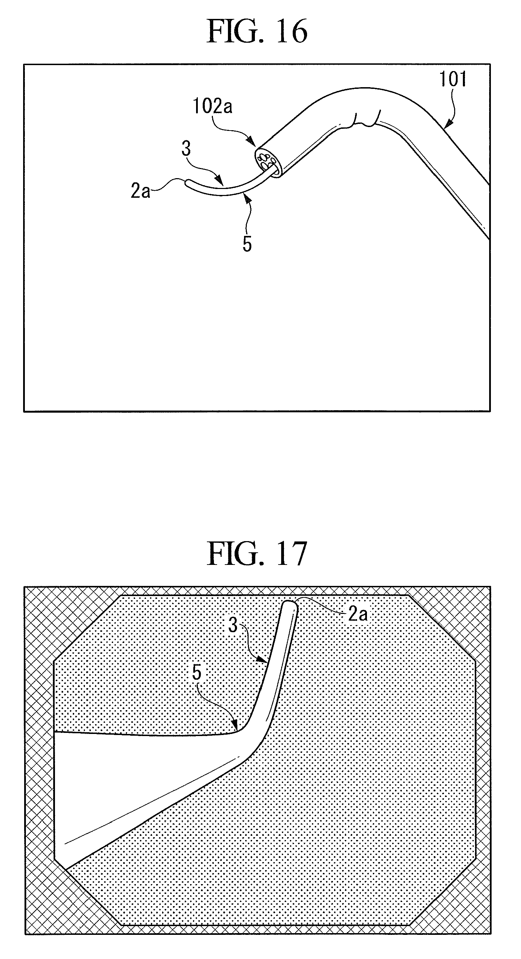

FIGS. 15 to 17 are views showing a fourth use state of the endoscope treatment system 100. FIG 15 is a front view showing the insertion section of the endoscope in the fourth use state. FIG. 16 is a photograph showing the endoscope in the fourth use state. FIG. 17 is an endoscope image in the fourth use state.

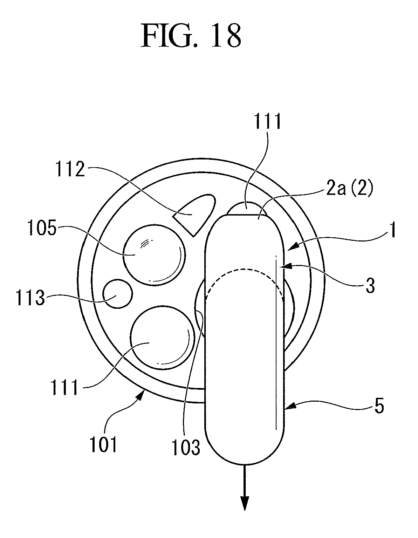

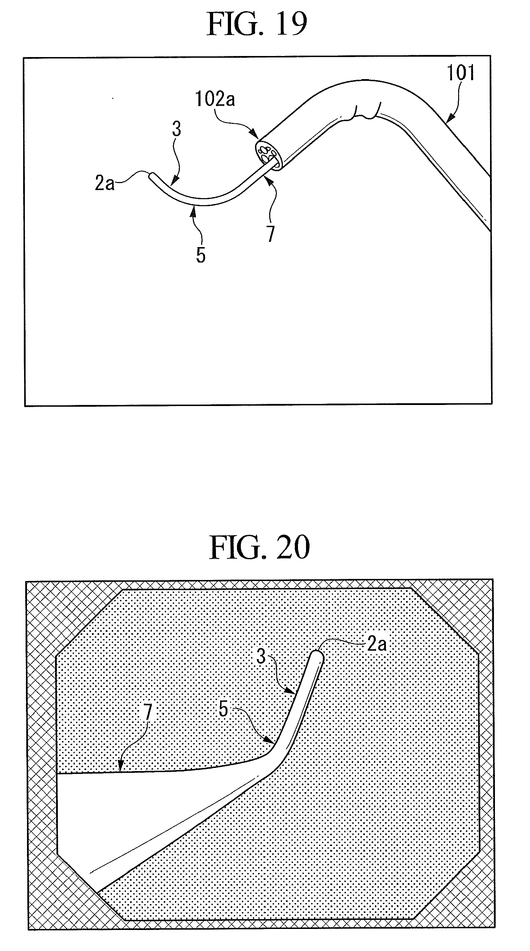

FIGS. 18 to 20 are views showing a fifth use state of the endoscope treatment system 100. FIG. 18 is a front view showing the insertion section of the endoscope in the fifth use state. FIG. 19 is a photograph showing the endoscope in the fifth use state. FIG. 20 is an endoscope image in the fifth use state.

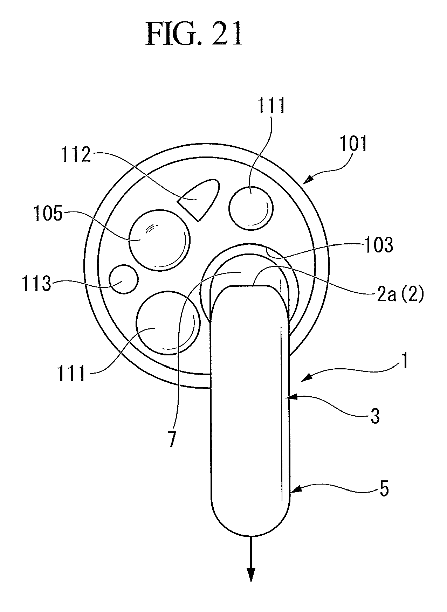

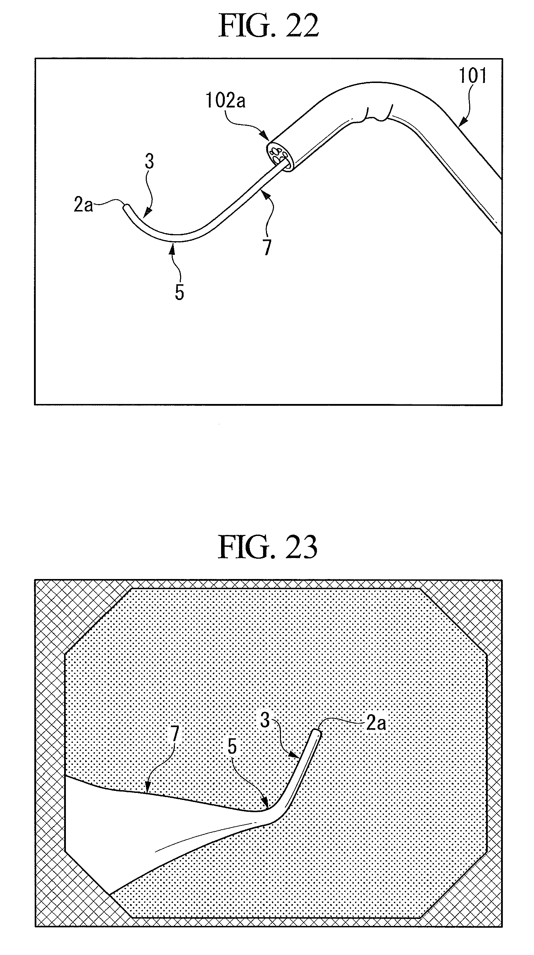

FIGS. 21 to 23 are views showing a sixth use state of the endoscope treatment system 100. FIG 21 is a front view showing the insertion section of the endoscope in the sixth use state. FIG. 22 is a photograph showing the endoscope in the sixth use state. FIG. 23 is an endoscope image in the sixth use state.

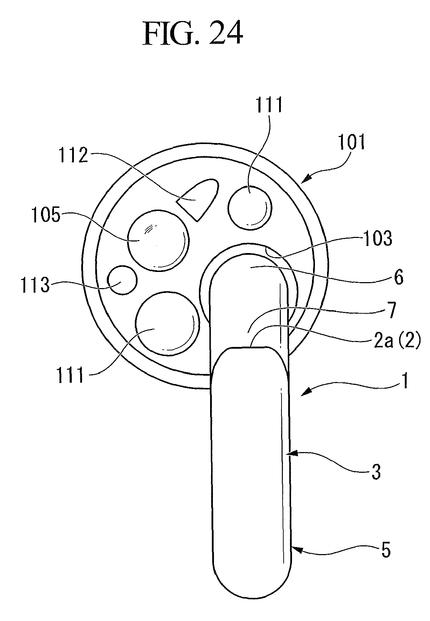

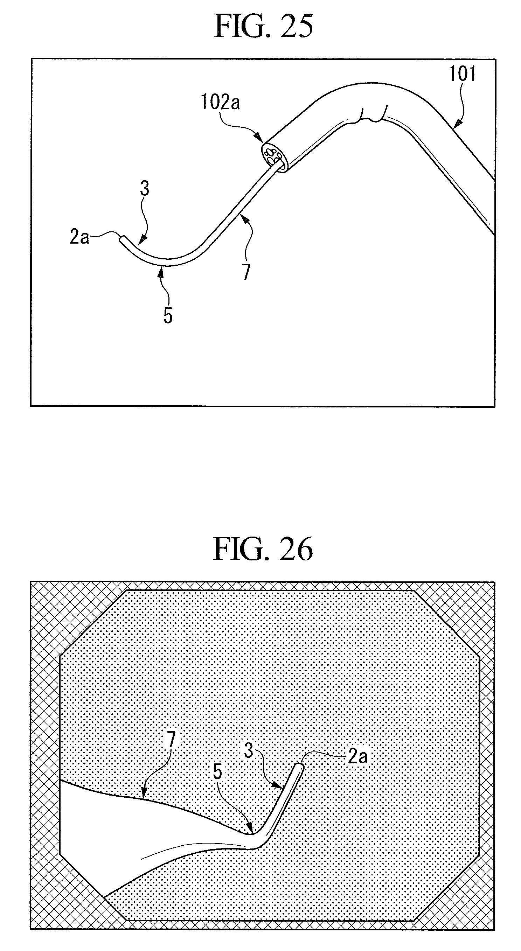

FIGS. 24 to 26 are views showing a seventh use state of the endoscope treatment system 100. FIG. 24 is a front view showing the insertion section of the endoscope in the seventh use state. FIG. 25 is a photograph showing the endoscope in the seventh use state. FIG. 26 is an endoscope image in the seventh use state.

Upon use of the endoscope treatment system 100, primarily, the operator connects a syringe S (a suction mechanism) to a proximal end of the longitudinal shaft member 2 (see FIG. 1). Accordingly, the liquid can be suctioned through the lumen 2c using the syringe S.

Next, the operator inserts the insertion section 102 of the endoscope 101 into the body of a patient, and guides the observation unit 105 of the distal end of the insertion section 102 to the treatment target. For example, as shown in FIG 4, the operator inserts the insertion section 102 of the endoscope 101 into the stomach G of the patient. The operator observes an image imaged by the observation unit 105 to identify a position of the treatment target.

As shown in FIG. 4, when the distal end of the insertion section 102 is guided to the treatment target, a curved state of the curve section 104 is held by the curve lock mechanism 110 (see FIG. 1) in a state curved at a predetermined curvature. As shown in FIG. 4, the curve section 104 is curved such that the channel 103 is disposed at an inner circumference side of the curve section 104.

Next, the operator inserts the endoscopic device 1 into the channel 103. The endoscopic device 1 is inserted into the channel 103 from a distal end 2a of the longitudinal shaft member 2.

The curved area 5 having a curved bias moves the inside of the curve section 104 of the endoscope 101 from the proximal end portion to the distal end portion. In the moving process, the curved area 5 is rotated about a central axis of the channel 103 in the channel 103 such that the curve direction coincides with a curve direction of the curve section 104 of the endoscope 101. The rotation of the curved area 5 is stopped at a position at which the curve direction of the curve section 104 coincides with the curve direction of the curved area 5.

From this state, the operator further inserts the longitudinal shaft member 2 into the channel 103. Accordingly, the distal end 2a of the longitudinal shaft member 2 protrudes from the distal end of the channel 103 (see FIGS. 4 and 5).

Here, as shown in FIGS. 6 and 7, the curved area 5 is curved such that the distal end 2a of the longitudinal shaft member 2 is curved from an optical axis L of the observation unit 105. As shown in FIG. 8, the distal end 2a side of the longitudinal shaft member 2 is reflected on an endoscope image imaged by the observation unit 105 of the endoscope 101. For this reason, the operator can recognize a position of the longitudinal shaft member 2 using the endoscope image.

Next, the operator further moves the longitudinal shaft member 2 toward the distal end of the channel 103. Accordingly, as shown in FIGS. 9 and 10, the curved area 5 is entirely exposed from the distal end of the channel 103. Here, the rotation guide section 6 (see FIG. 5) moves into the curve section 104 of the endoscope 101.

The rotation guide section 6 rotates the curved area 5 about the central axis of the channel 103 as a rotational center such that the curve direction of the curve section 104 coincides with the curve direction in the process of moving the rotation guide section 6 into the curve section 104 of the endoscope 101. The curved area 5 is rotated such that the treatment unit 3 approaches the optical axis of the observation unit 105.

Here, as shown in FIGS. 9, 12 and 15, the distal end 2a of the longitudinal shaft member 2 is rotated about the central axis of the channel 103.

As shown in FIGS. 10, 13 and 15, the longitudinal shaft member 2 is rotated while being pushed out of the distal end of the channel 103. Here, the distal end 2a of the longitudinal shaft member 2 is rotated and moved within a field of vision range of the observation unit 105.

For this reason, as shown in FIGS. 11, 14 and 17, the operator can recognize the position of the distal end 2a of the longitudinal shaft member 2 by the observation unit 105 always during rotation and movement of the distal end 2a of the longitudinal shaft member 2.

The rotation of the curved area 5 about the central axis of the channel 103 stops when the curve direction of the rotation guide section 6 coincides with the curve direction of the curve section 104. When the curve direction of the rotation guide section 6 coincides with the curve direction of the curve section 104, the distal end 2a of the longitudinal shaft member 2 is disposed at a position rotated 180° about the central axis of the channel 103.

After that, the operator further moves the longitudinal shaft member 2 toward the distal end of the channel 103. As a result, as shown in FIGS. 22 and 24, the distal end 2a of the longitudinal shaft member 2 is pushed out of the distal end of the channel 103. Accordingly, as shown in FIGS. 23 and 25, the operator can move the treatment unit 3 forward or rearward in a state in which a direction thereof is maintained, by advancing or retreating of the longitudinal shaft member 2 in the channel 103.

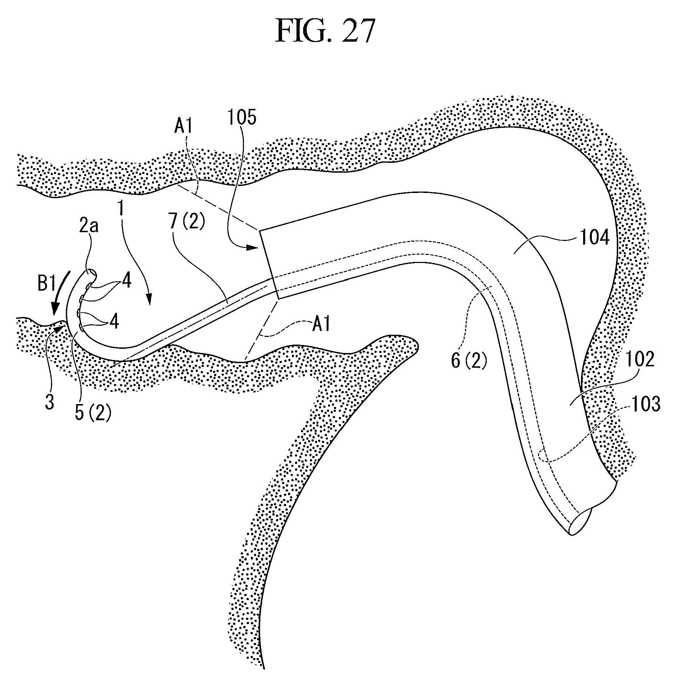

As shown in FIGS. 21 and 24, when the rotation guide section 6 of the longitudinal shaft member 2 is drawn out of the distal end of the channel 103, the curved area 5 of the longitudinal shaft member 2 is operated in a direction (a direction of an arrow B1 of FIG. 27) crossing the optical axis of the observation unit 105. The curved area 5 forms a recess in a wall surface of the treatment target by applying a pressing force in a vertical direction with respect to the wall surface (a pipe wall) of the treatment target using an operation thereof. As shown in FIG 27, when the curved area 5 forms the recess in the wall surface of the treatment target, the distal end 2a of the longitudinal shaft member 2 is disposed within a field of vision (reference sign A1) of the observation unit 105.

As the recess is formed in the wall surface of the treatment target by the operation of the curved area 5, a liquid such as a body fluid can be stored in the recess and the situation can be observed within the field of vision of the observation unit 105.

An operating direction of the curved area 5 of the longitudinal shaft member 2 when the rotation guide section 6 of the longitudinal shaft member 2 is drawn out of the distal end of the channel 103 (an operating direction of an operation of the curved area 5 spaced apart from the optical axis of the observation unit 105) coincides with the curve direction of the curve section 104 of the endoscope 101.

For this reason, as the curve operation of the curve section 104 of the endoscope 101 and the drawing operation of the rotation guide section 6 from the distal end of the channel 103 are combined, the wall surface of the treatment target can be pressed by the curved area 5 with a larger force.

According to the endoscopic device 1 and the endoscope treatment system 100 of the embodiment of the present invention, as the operator simply moves the longitudinal shaft member 2 of the endoscopic device 1 forward in the channel 103, the longitudinal shaft member 2 is rotated and an orientation of the curved area 5 is held. Here, the longitudinal shaft member 2 has a positional relationship in which the treatment unit 3 is proximate to the optical axis of the observation unit 105. For this reason, in the endoscope treatment system 100, the operator can easily recognize the position of the treatment unit 3 within the field of vision of the observation unit 105.

Next, a positional relationship between the curved area 5 and the rotation guide section 6 of the endoscopic device 1 will be described.

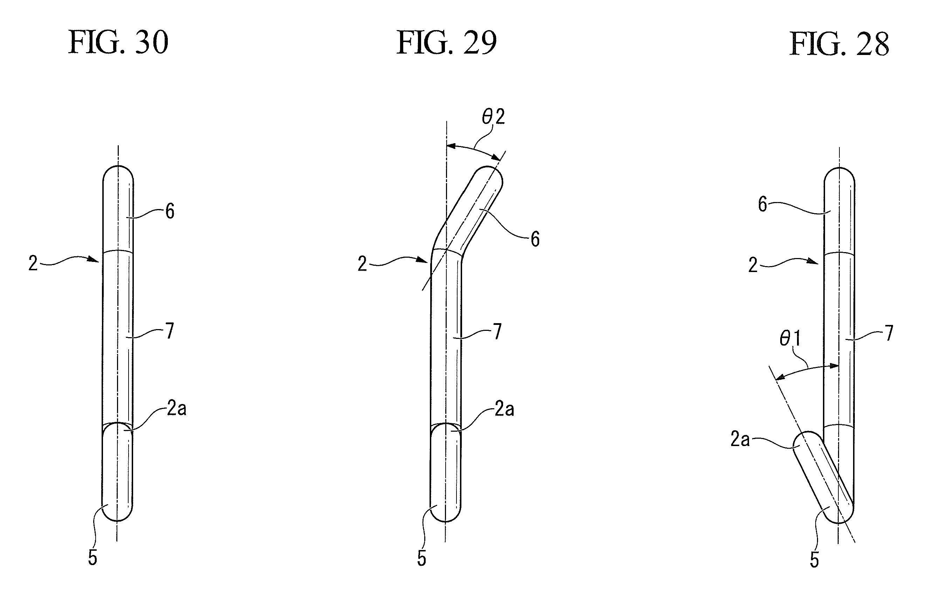

FIGS. 28 to 30 are views showing positional relationships between the curved area and the rotation guide section of the endoscopic device. FIG. 28 is a view showing Positional Relationship 1. FIG. 29 is a view showing Positional Relationship 2. FIG. 30 is a view showing Positional Relationship 3. FIGS. 28 to 30 are views taken in a direction of an arrow A of FIG. 3.

The positional relationship between the curved area 5 and the rotation guide section 6 can be arbitrarily set.

An angle θ1 between the curved area 5 and the linear section 7 and an angle θ2 between the linear section 7 and the rotation guide section 6 may each be optimized to set a direction of the distal end of the longitudinal shaft member 2 in a state in which the endoscopic device 1 is attached to the endoscope 101.

For example, as shown in FIGS. 28 to 30, Positional Relationship 1 to Positional Relationship 3 can be provided. Positional relationships different from Positional Relationship 1 to Positional Relationship 3 may be provided.

### (Positional Relationship 1)

As shown in Positional Relationship 1 of FIG. 28, the curved area 5 and the linear section 7 may be disposed in a direction deviated by the angle θ1.

### (Positional Relationship 2)

As shown in Positional Relationship 2 of FIG. 29, the linear section 7 and the rotation guide section 6 may be disposed in a direction deviated by the angle θ2.

### (Positional Relationship 3)

As shown in Positional Relationship 3 of FIG. 30, the angle θ1 between the curved area 5 and the linear section 7 and the angle θ2 between the linear section 7 and the rotation guide section 6 may be simultaneously 0°.

Next, modified examples of the endoscopic device will be described.

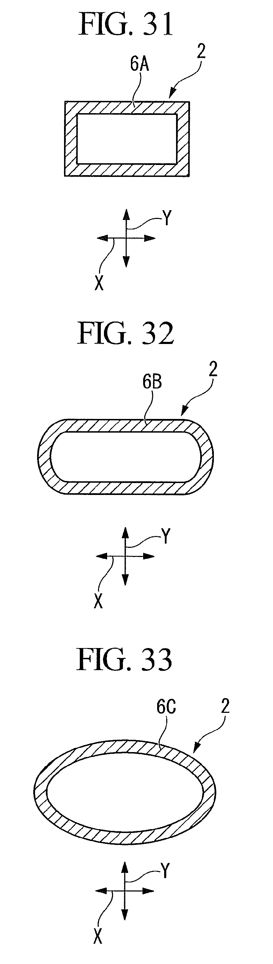

FIGS. 31 to 44 are cross-sectional views and perspective views showing the modified examples of the endoscopic device in the endoscope treatment system.

FIG 31 is a cross-sectional view showing Modified example 1 (a rotation guide section 6A) of the endoscopic device.

FIG. 32 is a cross-sectional view showing Modified example 2 (a rotation guide section 6B) of the endoscopic device.

FIG. 33 is a cross-sectional view showing Modified example 3 (a rotation guide section 6C) of the endoscopic device.

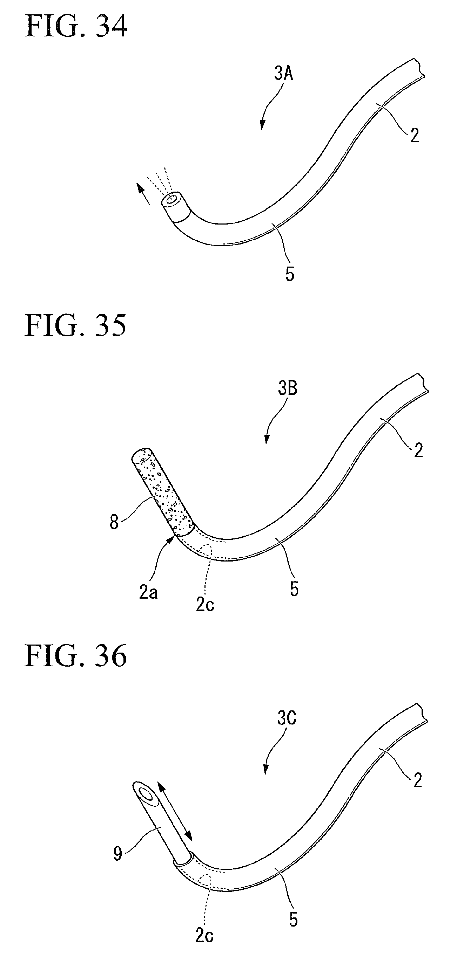

FIG. 34 is a perspective view showing Modified example 4 (a treatment unit 3A) of the endoscopic device.

FIG 35 is a perspective view showing Modified example 5 (a treatment unit 3B) of the endoscopic device.

FIG. 36 is a perspective view showing Modified example 6 (a treatment unit 3C) of the endoscopic device.

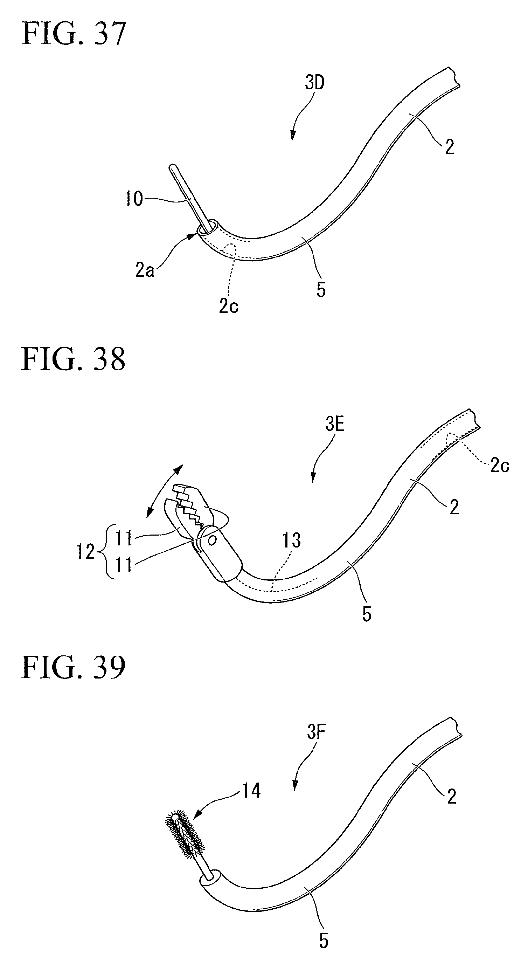

FIG. 37 is a perspective view showing Modified example 7 (a treatment unit 3D) of the endoscopic device.

FIG. 38 is a perspective view showing Modified example 8 (a treatment unit 3E) of the endoscopic device.

FIG. 39 is a perspective view showing Modified example 9 (a treatment unit 3F) of the endoscopic device.

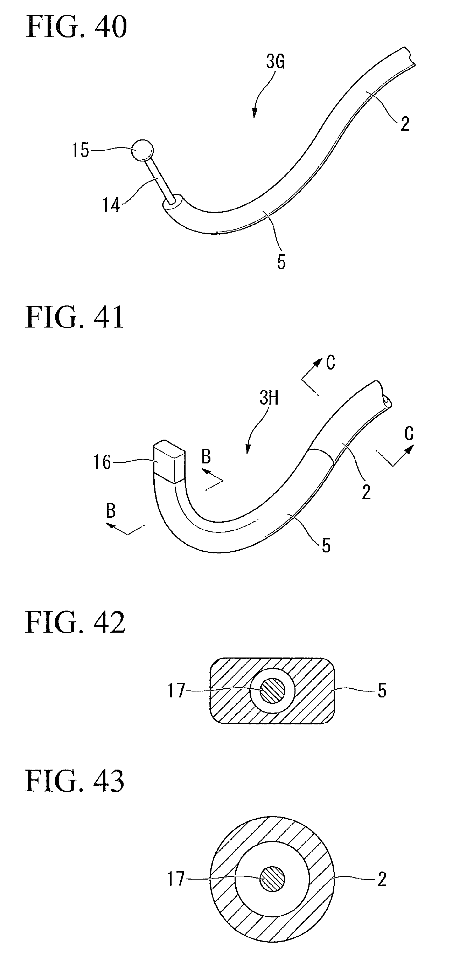

FIG. 40 is a perspective view showing Modified example 10 (a treatment unit 3G) of the endoscopic device.

FIG. 41 is a perspective view showing Modified example 11 (a treatment unit 3H) of the endoscopic device.

FIG. 42 is a cross-sectional view taken along line B-B of FIG. 41.

FIG. 43 is a cross-sectional view taken along line C-C of FIG. 41.

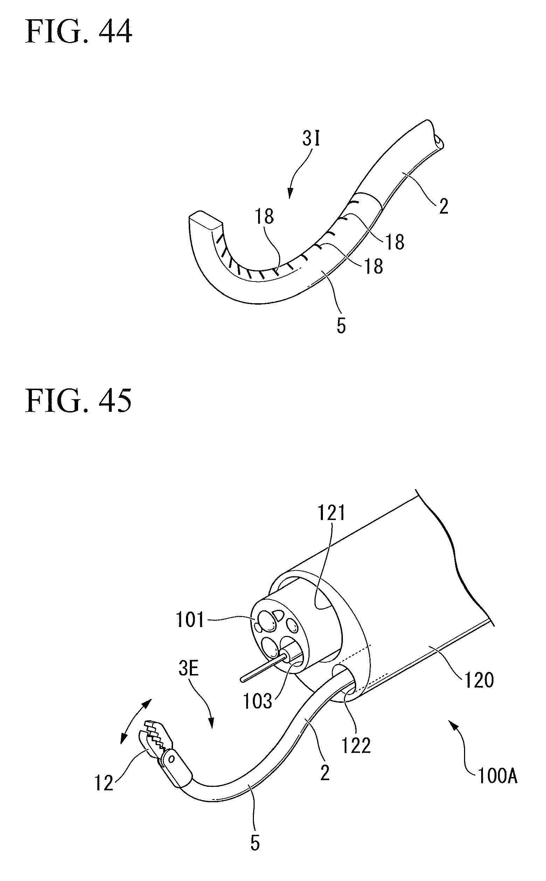

FIG. 44 is a perspective view showing Modified example 12 (a treatment unit 3I) of the endoscopic device.

### (Modified examples 1 to 3)

A shape of the rotation guide section 6 of the longitudinal shaft member 2 may be any one of Modified example 1 to Modified example 3, unlike the above-mentioned embodiment.

As shown in Modified example 1 of FIG. 31, the rotation guide section 6A of the longitudinal shaft member 2 has a rectangular outline in a cross-section perpendicular to a longitudinal axis of the longitudinal shaft member 2.

As shown in Modified example 2 of FIG. 32, the rotation guide section 6B of the longitudinal shaft member 2 has an oval outline in a cross-section perpendicular to the longitudinal axis of the longitudinal shaft member 2.

As shown in Modified example 3 of FIG 33, the rotation guide section 6C of the longitudinal shaft member 2 has an elliptical outline in a cross-section perpendicular to the longitudinal shaft of the longitudinal shaft member 2.

In Modified example 1 to Modified example 3, the rotation guide sections 6A, 6B and 6C have no curved bias. In the unloaded state, the rotation guide sections 6A, 6B and 6C have linear shapes.

For example, in the case of Modified example 1, the rotation guide section 6A of the longitudinal shaft member 2 is more easily curved in a direction in which a short side of a cross-section extends (a direction shown by reference sign Y of FIG. 31) than in a direction in which a long side of the cross-section extends (a direction shown by reference sign X of FIG. 31).

In Modified example 1 to Modified example 3, the same effect as of the endoscopic device 1 and the endoscope treatment system 100 described in the above-mentioned embodiment is exhibited.

In Modified example 1 to Modified example 3, the orientation of the distal end of the longitudinal shaft member 2 is more easily controlled than in the above-mentioned embodiment (in which the outline of the cross-section of the rotation guide section 6 is circular).

### (Modified example 4)

In Modified example 4, a configuration of the treatment unit 3A is different from that of the above-mentioned embodiment (the treatment unit 3).

As shown in FIG. 34, the treatment unit 3A is the same as in the above-mentioned embodiment in that the opening is provided in the distal end surface of the longitudinal shaft member 2. Meanwhile, the treatment unit 3A has no opening in the outer circumferential surface of the distal end of the longitudinal shaft member 2. The treatment unit 3A has a shape which is effective when liquid is supplied from the proximal end side toward the distal end side of the longitudinal shaft member 2 and the liquid from the opening of the distal end surface of the longitudinal shaft member 2 spreads to the treatment target.

### (Modified example 5)

In Modified example 5, a configuration of the treatment unit 3B is different from that of the above-mentioned embodiment (the treatment unit 3).

As shown in FIG 35, the treatment unit 3B has a porous suction member attached to the treatment unit 3A of the above-mentioned Modified example 2. A porous suction member 8 may be formed of, for example, a resin sponge, or the like. The treatment unit 3B can be appropriately used when the liquid in the body is suctioned from the distal end 2a of the longitudinal shaft member 2 toward a proximal end 2b (see FIG. 3) through the suction member 8 and the lumen 2c.

### (Modified example 6)

In Modified example 6, a configuration of the treatment unit 3C is different from that of the above-mentioned embodiment (the treatment unit 3).

As shown in FIG. 36, the treatment unit 3C is the same as in the above-mentioned embodiment in that the opening is formed in the distal end surface of the longitudinal shaft member 2. Meanwhile, the treatment unit 3C has no opening formed in the outer circumferential surface of the distal end of the longitudinal shaft member 2. The treatment unit 3C has a puncture needle 9 having a tubular shape disposed in the lumen 2c of the longitudinal shaft member 2. The puncture needle 9 has a sharp distal end and a proximal end extending to the proximal end of the longitudinal shaft member 2. The puncture needle 9 can freely advance and retreat in the lumen 2c of the longitudinal shaft member 2. A slider (not shown) configured to move the puncture needle 9 forward and rearward with respect to the longitudinal shaft member 2 may be installed at the proximal end of the longitudinal shaft member 2.

### (Modified example 7)

In Modified example 7, a configuration of the treatment unit 3D is different from that of the above-mentioned embodiment (the treatment unit 3).

As shown in FIG 37, the treatment unit 3D is the same as in the above-mentioned embodiment in that the opening is formed in the distal end surface of the longitudinal shaft member 2. Meanwhile, the treatment unit 3D has no opening formed in the outer circumferential surface of the distal end of the longitudinal shaft member 2. The treatment unit 3D has a needle electrode 10 disposed in the lumen 2c of the longitudinal shaft member 2. The distal end of the needle electrode 10 can protrude from the distal end 2a of the longitudinal shaft member 2.

### (Modified example 8)

In Modified example 8, a configuration of the treatment unit 3E is different from that of the above-mentioned embodiment (the treatment unit 3).

As shown in FIG. 38, the treatment unit 3E includes a forceps 12 in which a pair of forceps pieces 11 are installed to be opened and closed. A manipulation wire 13 configured to open and close the pair of forceps pieces 11 is connected to the pair of forceps pieces 11. The manipulation wire 13 is inserted into the lumen 2c of the longitudinal shaft member 2. The treatment unit 3E can be used as a grip forceps or a biopsy forceps.

### (Modified example 9)

In Modified example 9, a configuration of the treatment unit 3F is different from that of the above-mentioned embodiment (the treatment unit 3).

As shown in FIG. 39, the treatment unit 3F has a brush 14 configured to brush a surface of the tissue or the like. In the treatment unit 3F, the lumen 2c is not formed at the longitudinal shaft member 2, and the longitudinal shaft member 2 is a solid structure. The treatment unit 3F is fixed to the distal end of the longitudinal shaft member 2.

### (Modified example 10)

In Modified example 10, a configuration of the treatment unit 3G is different from that of the above-mentioned embodiment (the treatment unit 3).

As shown in FIG. 40, the treatment unit 3G has a rod-shaped electrode 16 having a spherical section 15 at a distal end thereof. In the treatment unit 3G, the lumen 2c is not formed at the longitudinal shaft member 2, and the longitudinal shaft member 2 is a solid structure. The treatment unit 3G is fixed to the distal end of the longitudinal shaft member 2.

In the treatment unit 3G, the distal end of the rod-shaped electrode 16 is spherical, which is a structure in which the inner surface of the channel 103 (see FIG. 1) cannot be easily damaged.

### (Modified example 11)

In Modified example 11, a configuration of the treatment unit 3H is different from that of the above-mentioned embodiment (the treatment unit 3).

As shown in FIGS. 41 to 43, the treatment unit 3H includes a rectangular parallelepiped electrode 16 installed at the distal end side of the longitudinal shaft member 2. The longitudinal shaft member 2 has a rectangular cross-section along an outline of the electrode 16 at a distal end side of the curved area 5, and is formed in a cylindrical shape nearer the proximal end side than the curved area 5. A wiring 17 configured to supply electric power to the electrode 16 is disposed in the longitudinal shaft member 2.

In the treatment unit 3H, a position of the electrode 16 can be adjusted by moving the longitudinal shaft member 2 forward and rearward in the channel 103 (see FIG. 1).

### (Modified example 12)

In Modified example 12, a configuration of the treatment unit 3I is different from that of the above-mentioned embodiment (the treatment unit 3).

As shown in FIG. 44, the treatment unit 3I includes a measurement unit 18 having a scale formed at the outer circumferential surface of the curved area 5. The measurement unit 18 can be appropriately used, for example, when a dimension of a tissue, which becomes a treatment target, is measured under the endoscope image. The scale of the measurement unit 18 may be formed of a material that can be recognized by, for example, an X-ray image.

In the treatment unit 3I, the longitudinal shaft member 2 is a solid structure, and a cross-section in the curved area 5 is a rectangular shape.

Next, a modified example of the endoscope treatment system will be described.

FIG. 45 is a perspective view showing a modified example (an endoscope treatment system 100A) of the endoscope treatment system.

In the endoscope treatment system 100A, the endoscopic device 1 is attached to an overtube 120 through which the endoscope 101 is inserted. That is, the endoscope treatment system 100A is different from the endoscope treatment system 100 in that the endoscopic device 1 is not attached to the endoscope 101.

A lumen 121 and a lumen 122 are formed at the overtube 120. The lumen 121 has a circular cross-section and can pass through the endoscope 101. The lumen 122 has a circular cross-section and can pass through the longitudinal shaft member 2.

In the above-mentioned configuration, the same effect as the above-mentioned embodiment (the endoscope treatment system 100) is exhibited.

In the endoscope treatment system 100A, when an active curving function is provided in the overtube 120, the same effect as in the above-mentioned embodiment (the endoscope treatment system 100) is exhibited even when the endoscope 101 is not provided.

In the endoscope treatment system 100A, even when an active curving function is not provided in the overtube 120, as the endoscope 101 inserted through the lumen 121 is provided in the curve section 104, the same effect as in the above-mentioned embodiment (the endoscope treatment system 100) is exhibited.

Hereinabove, while the exemplary examples of the present invention have been described, the present invention is not limited to these examples. Addition, omission, substitution and other modifications of components may be made without departing from the spirit of the present invention.

For example, when the rotation guide section configured as the longitudinal shaft member has a curved bias, the central axis of the rotation guide section and the central axis of the curved area may each be disposed on one of two intersecting planes.

In this case, a rotation direction of the curved area when the rotation guide section is inserted into the curve section of the endoscope is restricted to one direction.

In addition, a cross-sectional shape of the longitudinal shaft member at which the rotation guide section is disposed may be different from that of the other portion.

Further, the linear section may not be formed between the curved area and the rotation guide section.

### [Description of Reference Numerals]

- 1: endoscopic device
- 2: longitudinal shaft member
- 2a: distal end
- 2b: proximal end
- 2c: lumen
- 3, 3A-3I: treatment unit
- 4: opening
- 5: curved area
- 6, 6A-6C: rotation guide section
- 100,: 100A endoscope treatment system
- 101: endoscope
- 102: insertion section
- 102a: distal end
- 102b: proximal end
- 103: channel
- 104: curve section
- 105: observation unit
- 106: manipulation unit
- 109: power transmission member
- 110: curve lock mechanism

## Claims

1. An endoscopic device inserted into a channel in an endoscope, the endoscope including an observation unit being used to observe a treatment target, a curve section which is capable of being curvedly operated, an insertion section which is an elongated member having a distal end and a proximal end and in which the observation unit and the curve section are disposed at the distal end, the insertion section being inserted into a body, and a channel of an endoscope formed inside the insertion section, the endoscopic device comprising:
an elongated flexible longitudinal shaft member which is capable of freely advancing and retreating inside the channel;
a treatment unit installed at a distal end section of the longitudinal shaft member and performs a treatment of the treatment target;
a curved area which is a first portion of the longitudinal shaft member and being protruded from an opening of a distal end of the channel in a use state in which the longitudinal shaft member is inserted into the channel; and
a rotation guide section which is a second portion of the longitudinal shaft member and disposed at inside the channel and the curve section in a use state,
wherein a curve direction of the curved area and a curve direction of the rotation guide section are opposite to each other.

2. The endoscopic device according to Claim 1, wherein,
in a process in which the longitudinal shaft member is moved from a proximal end side to a distal end side of the longitudinal shaft member in the channel when the curve section is in a curved state,
the rotation guide section is rotated about a central axis of the channel as a rotational center,
the curved area is rotated such that the treatment unit approaches an optical axis of the observation unit by rotating the rotation guide section, and
an orientation of the curved area is held in a positional relationship in which the treatment unit is proximate to the optical axis in the use state.

3. The endoscopic device according to Claim 1,
wherein the curved area of the longitudinal shaft member is moved in a direction away from an optical axis of the observation unit by causing curvature in the curve section of the endoscope and being protruded the rotation guide section from the opening of the distal end of the channel.

4. The endoscopic device according to Claim 1,
wherein the rotation guide section is a curbed shape area of the longitudinal shaft member having a curved bias in a predetermined direction.

5. The endoscopic device according to Claim 1,
wherein the rotation guide section is an area in which at least a portion of the longitudinal shaft member is formed at one of a rectangular shape, an elliptical shape, and an oval shape of an outline of a cross-section perpendicular to the longitudinal shaft.

6. The endoscopic device according to Claim 1,
wherein a lumen is formed at the longitudinal shaft member along a longitudinal direction thereof,
an opening part which is communicated with the lumen of the longitudinal shaft member and allows a liquid in the body to enter the lumen, is formed at the treatment unit, and
a suction mechanism in which the liquid is moved from an inside the body to an outside of the body via the opening part and the lumen, is capable of connecting to the longitudinal shaft member.

7. An endoscope treatment system comprising an endoscope and an endoscopic device,
whrein the endoscope includes:
an observation unit being used to observe a treatment target;
a curve section which is capable of being curvedly operated;
an insertion section which is an elongated member having a distal end and a proximal end and in which the observation unit and the curve section are disposed at the distal end inserted into a body; and
a channel of an endoscope formed inside the insertion section,
the endoscopic device includes:
an elongated flexible longitudinal shaft member which is capable of freely advancing and retreating inside the channel;
a treatment unit installed at a distal end section of the longitudinal shaft member and performs a treatment of the treatment target;
a curved area which is a first portion of the longitudinal shaft member and being protruded from an opening of a distal end of the channel in a use state in which the longitudinal shaft member is inserted into the channel; and
a rotation guide section which is a second portion of the longitudinal shaft member and disposed at inside the channel and the curve section in a use state,
wherein a curve direction of the curved area and a curve direction of the rotation guide section are opposite to each other.

8. The endoscope treatment system according to Claim 7,
wherein, in a process in which the longitudinal shaft member is moved from a proximal end side to a distal end side of the longitudinal shaft member in the channel,
the rotation guide section is rotated about a central axis of the channel as a rotational center such that the treatment unit approaches an optical axis of the observation unit, and
an orientation of the curved area is held in a positional relationship in which the treatment unit is proximate to the optical axis in the use state.

9. The endoscope treatment system according to Claim 7,
wherein the endoscope comprises:
a manipulation unit disposed at a proximal end of the insertion section and configured to input a manipulation for curvedly operating the curve section;
a power transmission member connected to the manipulation unit and the curve section and configured to transmit a manipulation power from the manipulation unit to the curve section; and
a curve lock mechanism configured to hold the curve section in a curved state of a predetermined curvature,
wherein the rotation guide section is a curved shaped area of the longitudinal shaft member having a curved bias corresponding to the predetermined curvature.
